# EUROPÄISCHE PATENTANMELDUNG

(11) **EP 2 322 192 A1**
(43) Veröffentlichungstag der Anmeldung: **18.05.2011**
(21) Anmeldenummer: 09174293.2
(22) Anmeldetag: 28.10.2009
(51) Int. Cl.: A61K 36/13, A61K 36/61, A61K 36/48, A61K 36/534, A61K 36/53, A61P 3/10

(54) **Wirkstoffpräparat**

(71) Anmelder: NatureMed AS, 0188 Oslo (NO)
(72) Erfinder: Uz, Bilgi, 13000, Prag 3 Vinohrady (CZ)
(74) Vertreter: Tappe, Hartmut

(57) **Zusammenfassung**

Die Erfindung betrifft ein Wirkstoffpräparat zur äußeren Anwendung beim Menschen, umfassend eine Wirkstoff-Kombination aus zumindest einem Wirkstoff einer ersten Wirkstoffgruppe, umfassend Abies Alba, Eucalyptus Globulus und Glycine Soja, und mit einem weiteren Wirkstoff einer zweiten Wirkstoffgruppe umfassend Camphor, Thymus vulgaris, Menta pipperita und Urea.

## Beschreibung

Die vorliegende Erfindung betrifft ein Wirkstoffpräparat zur äußeren Anwendung beim Menschen, umfassend eine Wirkstoff-Kombination aus zumindest einem Wirkstoff einer ersten Wirkstoffgruppe, umfassend Abies Alba, Eucalyptus Globulus und Glycine Soja und einem weiteren Wirkstoff einer zweiten Wirkstoffgruppe, umfassend Camphor, Thymus vulgaris, Menta pipperita und Urea.

Des Weiteren betrifft die vorliegende Erfindung die Verwendung des Wirkstoffpräparats zur Herstellung eines zur Behandlung von Menschen geeigneten Präparats, insbesondere zur Behandlung von Diabetes.

Die möglichen positiven Effekte einer äußeren Anwendung von pflanzlichen Extrakten bzw. Wirkstoffpräparaten auf pflanzlicher Basis auf den menschlichen Organismus sind lange bekannt. Dabei lassen sich bestimmten pflanzlichen Extrakten bestimmte Wirkungen zuordnen. Aufgabe der vorliegenden Erfindung ist es, ein Wirstoffpräparat basierend auf pflanzlichen Wirkstoffen bereitzustellen, dass sich insbesondere zur Anwendung bei an Diabetes leidenden Menschen eignet.

Zur Lösung dieser Aufgabe weist die vorliegende Erfindung die Merkmale des Anspruchs 1 oder 7 auf.

In Versuchen mit Kombinationen aus den vorstehend genannten Wirkstoffen, die auch umgangssprachlich bekannt sind unter den Begriffen Weißtannenextrakt (Abies Alba), Eukalyptus Globuli (Eukalyptus Globulus), Sojabohnen (Glycine Soja), Camphor, Thymian (Thymus vulgaris), Pfefferminze (Menta pipperita) und Harnstoff (Urea), hat sich gezeigt, dass bereits bei äußerlicher Anwendung positive Effekte feststellbar sind; insbesondere bei unter Diabetes leidenden Menschen.

Besonders nachhaltige Effekte, die in einem unmittelbarem Zusammenhang zwischen der äußeren Anwendung der erfindungsgemäßen Wirkstoffkombination an einem unter Diabetes leidenden Menschen und dem Einfluss dieser äußeren Anwendung auf den Blutzuckerspiegel deutlich werden, sind bei einer Ausführung des Wirkstoffpräparats mit einer Wirkstoff-Kombination feststellbar, die Abies Alba, Eucalyptus Globulus, Glycine Soja, Abies Alba, Eucalyptus Globulus und Glycine Soja umfasst.

Selbst bei verhältnismäßig geringer Dosierung der Wirkstoffkombination bzw. relativ kleiner, mit der Wirkstoffkombination beaufschlagter Oberfläche des menschlichen Körpers konnte nach Zugabe einer Aminosäure zu der Wirkstoffkombination ein deutlicher Effekt festgestellt werden, da offensichtlich die Ergänzung der Wirkstoffkombination mit einer Aminosäure eine durch die Aminosäure bedingte katalytische Wirkung zeigt. Als einsetzbare Aminosäuren haben sich als besonders vorteilhaft herausgestellt, eine oder mehrere Aminosäuren aus einer Gruppe von Aminosäuren, die Methionin, Methyl Aspartic Acid und Methyl Glutamic Acid umfasst.

Um eine einfache Applikation des Wirkstoffpräparats bzw. der in dem Wirkstoffpräparat enthaltenen Wirkstoffkombination zu ermöglichen, ist es vorteilhaft, wenn das Wirkstoffpräparat als Lösungs- und/oder Konservierungsmittel einen Alkohol aufweist, der vorzugsweise aus einer Gruppe von Alkoholen gewählt ist, die Cetyl Alkohol, Ceteareth-25, Ceteareth-6 und Stearylalcohol umfasst.

Um eine die Anwendung des Wirkstoffpräparats am menschlichen Körper erleichternde, anhaftende Applikation des Wirkstoffpräparats am menschlichen Körper zu ermöglichen, ist es vorteilhaft, wenn das Wirkstoffpräparat auf Basis einer Wasser/Öl-Emulsion hergestellt ist, die ein pflanzliches Verdickungsmittel aufweist. Zum einen wird aufgrund der durch das Verdickungsmittel erhöhten Viskosität die Haftung des Wirkstoffpräparats an einer ausgewählten Körperstelle erhöht, zum anderen wird diese Viskositätserhöhung erzielt, ohne dass synthetische Zusatzmittel hinzugegeben werden müssten.

Als pflanzliche Verdickungsmittel haben sich dabei Hennamehl oder Getreidemehl als besonders vorteilhaft herausgestellt.

Zur Erzeilung einer cremig-pastösen Erscheinungsform des Wirkstoffpräparats kann das Wirkstoffpräparat zumindest einen Zusatzstoff aus einer Gruppe von Zusatzstoffen, umfassend Glycerin, Paraffinum Liquidum, Glyceryl Stearate (Wollwachs) oder auch Dimethycone (Silikon) aufweisen. Diese cremig-pastöse Konsistenz ermöglicht eine entsprechend langwährende Beaufschlagung ein und derselben Körperstelle mit dem Wirkstoffpräparat, so dass dieses auch ohne Zuhilfenahme von weiteren Trägermaterialien, wie beispielsweise Verbandmaterial, lagefixiert an Ort und Stelle verbleiben kann.

### Ausführungsbeispiel:

Als besonders für die Praxis geeignet und vielseitig anwendbar hat sich eine Rezeptur für das Wirkstoffpräparat herausgestellt, die in nachfolgender Tabelle mit den einzelnen aufgeführten Rezepturbestandteilen dargestellt ist.

| | |
|---|---|
| - Lawsonia Inermis | 16,60% |
| - Glycerin | 5% |
| - Paraffinum Liquidum | 5% |
| - Benzophenone - 4 | 4% |
| - Glyceryl Stearate | 4% |
| - Cetyl Alkohol | 4% |
| - Ceteareth - 25 | 2% |
| - Ceteareth - 6 and Stearylalcohol | 2% |
| - Dimethycone | 1% |
| - Abies Alba | 1% |
| - Eucalyptus Globulus | 1% |
| - Glycine Soja | 0,5% |
| - Methylparaben | 0,1% |
| - Propylparaben | 0,1% |
| - Isopropylparaben | 0,1% |
| - Butylparaben | 0,1% |
| - Methylchloroisothiazolinone, methyllisothiazolinone | 0,1% |
| - Parfum | 0,1% |
| - Alcohol | 0,1% |
| - Sodium Chloride | 0,1% |
| - Methionin | 0,1% |
| - Potassium Chloride | 0,05% |
| - Calcium Chloride | 0,05% |
| - Camphor | 0,05% |
| - Thymus vulgaris | 0,05% |
| - Menta pipperita | 0,05% |
| - Urea | 0,05% |
| - Methyl Aspartic Acid | 0,05% |
| - Methyl Glutamic Acid | 0,05% |
| - Rest Wasser | |

Bei dem explizit hinsichtlich seiner Zusammensetzung gelisteten Ausführungsbeispiel wird als Verdickungsmitte Hennamehl (Lawsonia Inermis) verwendet. Ergänzend zu den bereits vorstehend erläuterten vorteilhaften Wirkstoffen und Wirkstoffkatalysatoren (Aminosäuren) kommen bei der beispielhaft ausgewählten Rezeptur zusätzliche Bestandteile zum Einsatz, die wie beispielsweise das Benzophenone-4, das als Lichtschutz wirkt, eine Anwendung des Wirkstoffpräparats auch unter direkter Sonnenbestrahlung, als außerhalb geschlossener Räume, ermöglicht, ohne dass eine negative Beeinflussung der Wirkstoffkombination durch UV-Strahlung zu befürchten wäre. Weiterhin finden sich in der beispielhaften Rezeptur zusätzliche Zuschlagstoffe als Bestandteile, wie beispielsweise Potassium Chlorid (Pottasche) und verschiedene Salze.

Je nach gewünschter Haltbarkeit des Wirkstoffpräparats können diesem auch konservierende Zusätze, wie beispielsweise Propylparaben oder Methylchloroisothiazolinone, hinzugefügt werden. Auch kann sich der ergänzende Zusatz von antiseptisch wirkenden Bestandteilen als vorteilhaft erweisen. Als Füllstoff bzw. zur Einstellung der gewünschten Konzentration des Wirkstoffpräparats besteht der verbleibende Rest der Gesamtmasse des Wirkstoffpräparats im Wesentlichen aus Wasser.

Das erfindungsgemäße Wirkstoffpräparat eignet sich insbesondere im Fall des vorstehend erläuterten Rezepturbeispiels zum Auftragen auf die Körperoberfläche in unterschiedlichen Körperregionen. Insbesondere sind dabei die Innenflächen der Hände, die Fußsohlen, die Kniegelenke sowie der gesamte Rückenbereich hervorzuheben.

## Patentansprüche

1. Wirkstoffpräparat zur äußeren Anwendung beim Menschen, umfassend eine Wirkstoff-Kombination aus zumindest einem Wirkstoff einer ersten Wirkstoffgruppe, umfassend Abies Alba, Eucalyptus Globulus und Glycine Soja, und mit einem weiteren Wirkstoff einer zweiten Wirkstoffgruppe umfassend Camphor, Thymus vulgaris, Menta pipperita und Urea.

2. Wirkstoffpräparat nach Anspruch 1,
**dadurch gekennzeichnet,**
**dass** die Wirkstoff-Kombination Abies Alba, Eucalyptus Globulus, Glycine Soja, Abies Alba, Eucalyptus Globulus und Glycine Soja umfasst.

3. Wirkstoffpräparat nach Anspruch 1 oder 2,
**dadurch gekennzeichnet,**
**dass** das Wirkstoffpräparat neben der Wirkstoff -Kombination zumindest eine Aminosäure aus einer Gruppe von Aminosäuren, umfassend Methionin, Methyl Aspartic Acid und Methyl Glutamic Acid, aufweist.

4. Wirkstoffpräparat nach einem der vorangehenden Ansprüche,
**dadurch gekennzeichnet,**
**dass** das Wirkstoffpräparat als Lösungs- und/oder Konservierungsmittel zumindest einen Alkohol aus einer Gruppe von Alkoholen, umfassend Cetyl Alkohol, Ceteareth-25, Ceteareth-6 und Stearylalcohol, aufweist.

5. Wirkstoffpräparat nach einem der vorangehenden Ansprüche,
**dadurch gekennzeichnet,**
**dass** das Wirkstoffpräparat auf Basis einer Wasser/Öl-Emulsion hergestellt ist, die ein pflanzliches Verdickungsmittel aufweist.

6. Wirkstoffpräparat nach Anspruch 5,
**dadurch gekennzeichnet,**
**dass** das Wirkstoffpräparat zur Erzielung einer cremig-pastösen Konsistenz zumindest einen Zusatzstoff aus einer Gruppe von Zusatzstoffen, umfassend Glycerin, Paraffinum Liquidum, Glyceryl Stearate und Dimethycone, aufweist.

7. Verwendung des Wirkstoffpräparats nach einem oder mehreren der Ansprüche 1 bis 6 zur Herstellung eines zur Behandlung von Menschen geeigneten Präparats, insbesondere zur Behandlung von Diabetes.
